# FASCICULE DE BREVET EUROPEEN

(11) **EP 3 232 916 B1**
(45) Date de publication et mention de la délivrance du brevet: **02.08.2023**
(21) Numéro de dépôt: 15823689.3
(22) Date de dépôt: 18.12.2015
(51) Int. Cl.: A61B 5/00, A61B 5/11

(54) **SYSTEME DE CONTROLE DU MOUVEMENT CYCLIQUE D'UN SEGMENT CORPOREL D'UN INDIVIDU**
SYSTEM ZUR STEUERUNG DER ZYKLISCHEN BEWEGUNG EINES KÖRPERSEGMENTS EINER PERSON
SYSTEM FOR CONTROLLING THE CYCLIC MOTION OF A BODY SEGMENT OF AN INDIVIDUAL

(30) Priorité: 18.12.2014 FR 1462735
(43) Date de publication de la demande: 25.10.2017
(73) Titulaire: UNIVERSITE GRENOBLE ALPES, 38400 Saint Martin d'Hères (FR); Centre Hospitalier Universitaire Grenoble, 38700 La Tronche (FR); Centre National de la Recherche Scientifique (CNRS), 75016 Paris (FR)
(72) Inventeur: CINQUIN, Philippe, 38330 St Nazaire les Eymes (FR); NOUGIER, Vincent, 38330 Saint Ismier (FR); GRIFFET, Jacques, 38240 Meylan (FR); COURVOISIER, Aurélien, 38000 Grenoble (FR); STRUBER, Lucas, 38450 Vif (FR); DUBOUSSET, Jean, 75013 Paris (FR)
(74) Mandataire: Novagraaf Technologies
(86) Numéro de dépôt international: PCT/FR2015/053653
(87) Numéro de publication internationale: WO 2016/097655

(56) Documents cités:
- WO-A1-2008/129442
- US-A1- 2006 184 336
- US-A1- 2009 204 030
- US-A1- 2011 063 114
- US-A1- 2013 184 611
- US-A1- 2013 207 889

## Description

### DOMAINE DE L'INVENTION

La présente invention concerne un système de contrôle d'un mouvement cyclique d'un segment corporel d'un individu.

### ARRIERE PLAN DE L'INVENTION

L'analyse et la correction du mouvement d'un segment corporel d'un individu est nécessaire dans différentes applications biomédicales, notamment la rééducation.

Ainsi, différentes pathologies sont susceptibles d'altérer des mouvements ou des postures d'un individu, qu'il est donc nécessaire d'adapter et/ou de corriger. Par un contrôle du mouvement ou de la posture, il est possible de remédier à ou tout au moins de limiter les altérations dues à la pathologie. A cet effet, ces mouvements et postures sont généralement réalisés sous le contrôle d'un kinésithérapeute ou d'un autre spécialiste.

Cependant, hors de la surveillance d'un thérapeute, l'individu peut à nouveau adopter des postures ou des mouvements erronés, ce qui nuit à la qualité et à la rapidité de la rééducation.

Il serait donc souhaitable de procurer à l'individu un moyen de contrôler ses mouvements et postures en-dehors des séances de rééducation proprement dites et en l'absence d'un thérapeute.

En ce qui concerne la correction de la posture, il existe des systèmes permettant de comparer une posture donnée à une posture de référence et de fournir, par une rétroaction, une information à l'individu sur la conformité de sa posture [1]-[4]. Cependant, ces deux solutions permettent d'étudier un nombre fini de postures mais ne donnent aucune indication sur la qualité du mouvement permettant de passer de l'une à l'autre de ces postures. Or, il existe différents mouvements pour parvenir à une posture finale, dont certains peuvent être néfastes sur le plan physiologique. Par conséquent, la seule vérification d'une posture correcte n'est pas suffisante pour vérifier que le mouvement ayant conduit à cette posture a été également correctement réalisé.

Il existe par ailleurs des systèmes de capture du mouvement destinés à recréer le mouvement d'un individu sur un ordinateur, par exemple à des fins d'animation. Cependant, ces systèmes ne permettent pas d'analyser en temps réel la qualité du mouvement ni de fournir à l'individu une information sur un éventuel mouvement incorrect.

Le document [5] décrit un vêtement auquel sont intégrés des capteurs et des actionneurs permettant de corriger en temps réel les mouvements d'un individu. Un mouvement de référence, correspondant à un mouvement idéal d'un individu normal, est enregistré au préalable dans le système. Le mouvement de l'individu porteur du vêtement est comparé par rapport à ce mouvement de référence, et, si un écart significatif est mesuré, traduisant un mouvement erroné, les actuateurs sont actionnés de sorte à corriger en temps réel le mouvement de l'individu. Cependant, avec un tel système, l'individu reste passif lors de la correction du mouvement par les actuateurs, ce qui ralentit sa progression vers un mouvement optimisé. Par ailleurs, le mouvement de référence étant externe à l'individu, il reste possible que les capacités physiques de l'individu ne soient pas adaptées à la réalisation de ce mouvement idéal.

Le document [6] décrit un procédé de contrôle d'un mouvement réalisé par un individu dans le cadre d'un exercice de rééducation. A cet effet, un mouvement idéal est préalablement réalisé par un thérapeute équipé d'un ou plusieurs capteurs tridimensionnels de mouvement et enregistré dans la mémoire d'un processeur. L'individu est ensuite équipé desdits capteurs et réalise le mouvement souhaité, qui est enregistré et comparé au mouvement idéal. Une rétroaction est fournie à l'individu sous la forme d'un écart visualisable entre le mouvement réalisé et le mouvement idéal. Cependant, la comparaison n'est effectuée qu'une fois que le mouvement a été entièrement réalisé. Par conséquent, il ne permet pas à l'individu de modifier l'exécution du mouvement pour le corriger dès qu'un tel écart significatif par rapport au mouvement idéal se produit, en cours d'exécution. En outre, ce procédé est destiné au suivi d'exercices de rééducation ou d'entraînement sportif effectués par l'individu, de sorte qu'il n'est mis en oeuvre qu'à des moments spécifiques de la vie de l'individu.

Or, pour augmenter l'efficacité du suivi, il est souhaitable de pouvoir contrôler un mouvement d'un individu dans sa vie quotidienne, notamment s'agissant des mouvements cycliques tels que la marche.

Le document US 2013/0207889 A1 divulgue un système de contrôle de la posture d'un individu.

Le document WO 2008/129442 A1 décrit un système de contrôle de mouvement adapté pour des personnes handicapées.

Le document US 2011/063114 A1 divulgue un système de contrôle de la posture d'un individu.

Le document US 2006/184336 A1 concerne la capture et le contrôle de mouvement selon plusieurs axes ou degrés de liberté.

Le document US 2009/204030 A1 concerne l'analyse de mouvements de personnes présentant des paralysies latérales (hémiplégie). L'utilisation fonctionnelle d'un membre est évaluée en déterminant la synchronicité des mouvements du patient en fonction de la cyclicité du mouvement. La progression de la rééducation peut être surveillée et des informations peuvent être fournies aux thérapeutes et aux patients sur l'état du processus de rééducation. Il est également possible de donner un feedback au patient (ou au thérapeute) en temps réel, c'est-à-dire pendant que certains mouvements sont effectués. Le dispositif décrit comprend un capteur de mouvement, un moyen de fixation dudit capteur, un dispositif de rétroaction et un processeur configuré pour enregistrer un mouvement cyclique de référence propre à l'individu, détecter à partir de données de mesure du capteur un mouvement cyclique d'un individu, le comparer avec le mouvement cyclique de référence, et déclencher une rétroaction en fonction du résultat de cette comparaison.

### BREVE DESCRIPTION DE L'INVENTION

Un but de l'invention est de remédier aux inconvénients des systèmes existants et de permettre à l'individu d'améliorer de manière plus efficace la réalisation d'un mouvement cyclique déterminé.

Par « mouvement » d'un segment, on entend un déplacement dudit segment et éventuellement une déformation dudit segment.

Par « mouvement cyclique » on entend dans le présent texte un mouvement réalisé par l'individu de manière répétitive et prédictible, c'est-à-dire que ledit mouvement est réalisé plusieurs fois selon un rythme déterminé. A titre d'exemple, la marche, la course, la nage, l'action de ramer, de monter ou descendre des escaliers, un travail à la chaîne ou plus généralement tout mouvement répétitif ou répété plusieurs fois consécutives sont des mouvements cycliques au sens de la présente invention.

Par « paramètre du mouvement » on entend tout paramètre permettant de qualifier et/ou de quantifier le mouvement à un instant donné pendant son exécution, sur une partie du mouvement ou dans son ensemble. Il peut s'agir par exemple de l'orientation, vitesse, accélération, amplitude de déplacement, etc. dudit segment ou même bien sûr du mouvement dans son ensemble.

A cet effet, un but de l'invention est de permettre l'analyse du mouvement cyclique d'au moins un segment corporel d'un individu et de restituer à l'individu une information en temps réel sur la conformité de ce mouvement vis-à-vis d'un mouvement de référence, en vue de permettre à l'individu de corriger lui-même son mouvement. Un autre but de l'invention est de tenir compte des capacités propres à l'individu, en tenant compte de sa progression au cours du temps, sans se baser sur une référence externe à l'individu.

L'invention est définie par la revendication indépendante 1 annexée. Des modes de réalisation préférés du système revendiqué sont définis par les revendications dépendantes 2-13.

### BREVE DESCRIPTION DES DESSINS

D'autres caractéristiques et avantages de l'invention ressortiront de la description détaillée qui va suivre, en référence aux dessins annexés sur lesquels :
- les figures 1A à 1C illustrent différentes étapes de mise en oeuvre du contrôle d'un mouvement cyclique chez un individu,
- les figures 2A et 2B sont respectivement une vue de face et de dos d'un vêtement destiné à couvrir le haut du corps d'un individu, selon un mode de réalisation de l'invention,
- les figures 3A et 3B sont respectivement une vue de face et de dos d'un vêtement destiné à couvrir le bas du corps d'un individu, selon un mode de réalisation de l'invention.

### DESCRIPTION DETAILLEE DE L'INVENTION

Le système de contrôle du mouvement cyclique d'au moins un segment corporel d'un individu comprend au moins un capteur tridimensionnel destiné à mesurer des mouvements du segment corporel à étudier.

Le système comprend en outre un moyen pour fixer chaque capteur à chaque segment corporel considéré.

Tout moyen de fixation approprié peut être employé, tel que de la colle, un ruban adhésif, ou encore un vêtement destiné à être porté par l'individu et dont une partie est destinée à être ajustée étroitement au segment à contrôler. Par « vêtement » on entend dans le présent texte toute pièce d'habillement susceptible de recouvrir au moins une partie du corps de l'individu : les membres, y compris leurs extrémités (pieds, mains), le tronc, la tête.

A cet effet, le vêtement est avantageusement réalisé en un matériau textile (tissu, tricot, non tissé, etc.) extensible.

La nature des fibres du matériau textile est déterminée par l'homme du métier en fonction de l'application.

De manière alternative ou complémentaire, le vêtement peut également comprendre des moyens de fixation vis-à-vis du segment considéré, tels que des lacets dans le cas où le vêtement est une chaussure, par exemple.

Le capteur tridimensionnel est situé dans une région du vêtement destinée à s'ajuster sur ledit segment lorsque le vêtement est porté par l'individu. Ainsi, le capteur est rendu solidaire dudit segment, sans qu'il soit nécessaire d'employer un moyen de fixation direct (par exemple de la colle) sur ledit segment. Le capteur est donc toujours positionné au même endroit du segment même après plusieurs habillages et déshabillages.

Par « capteur tridimensionnel » on entend dans le présent texte un capteur adapté pour fournir des informations sur la position et l'orientation dans l'espace d'un segment corporel auquel il est attaché.

De manière avantageuse, on utilise comme capteur tridimensionnel une centrale inertielle, mais d'autres technologies de capteurs sont envisageables, tels que des élastomères conducteurs et les fibres optiques. En effet, des capteurs utilisant des élastomères conducteurs [7] ou des fibres optiques [8] ont déjà été utilisés pour mesurer des angles articulaires. Les élastomères possèdent des propriétés piézorésistives, c'est-à-dire que leur déformation modifie le courant électrique qui les traverse et les fibres optiques ont une courbure proportionnelle au débit lumineux les traversant. En utilisant un réseau de fibres optiques ou d'élastomères il est donc possible d'obtenir une orientation 3D d'un segment par rapport à un autre.

De manière avantageuse, plusieurs capteurs solidaires d'un même segment permettent de rendre compte plus précisément du mouvement dudit segment, notamment lorsque le mouvement à exécuter est complexe ; cependant, dans certains cas, l'utilisation d'un seul capteur pour un segment peut être suffisante, par exemple lorsque le mouvement à effectuer est peu variable ou est réalisé dans un plan.

Par ailleurs, le système peut comprendre des capteurs destinés à être solidarisés à différents segments du corps lorsque le mouvement implique plusieurs segments. Par exemple, pour contrôler le mouvement d'un membre, on intègre à un vêtement des capteurs solidaires des différents segments composant le membre, et éventuellement au moins un capteur solidaire du tronc de l'individu.

Le système comprend par ailleurs au moins un processeur couplé au capteur - ou à la pluralité de capteurs le cas échéant - la liaison pouvant être filaire ou sans fil. Le processeur est solidaire de l'individu, par exemple lorsque ledit processeur est agencé dans ou sur un vêtement. De manière alternative, le processeur d'un smartphone ou tout autre dispositif portable susceptible d'être embarqué sur l'individu peut être utilisé pour effectuer les calculs.

Le processeur est configuré pour enregistrer le mouvement de l'individu en intégrant les données de mesure du ou des capteur(s) tridimensionnel(s) et pour le comparer en temps réel à un mouvement de référence. A cet effet, le processeur met en oeuvre une comparaison de signaux (à savoir un ou plusieurs signaux représentatifs du mouvement en cours de réalisation et un ou plusieurs signaux représentatifs du mouvement de référence). Les algorithmes de comparaison de signaux points par points entre des bornes extrêmes sont classiques en eux-mêmes et ne seront pas décrits en détail ici. Cette comparaison est réalisée en temps réel pour rendre compte des aspects dynamiques du mouvement et de son organisation temporelle.

Lorsque plusieurs capteurs sont utilisés, le processeur met en oeuvre un algorithme de fusion de données pour tenir compte des données de mesure des différents capteurs.

De manière particulièrement avantageuse, ledit mouvement de référence n'est pas défini a priori, indépendamment de l'individu, mais est au contraire enregistré préalablement sur l'individu au moyen des capteurs, sous le contrôle d'un thérapeute ou d'un autre spécialiste. Ainsi, le mouvement de référence est fonction de l'individu et de ses capacités motrices ; il ne correspond pas à un mouvement idéal dans l'absolu mais à un mouvement considéré comme optimal pour l'individu en question.

Pour enregistrer ce mouvement de référence dans la mémoire du système, l'individu équipé des capteurs effectue le mouvement plusieurs fois sous le contrôle du thérapeute, qui enregistre plusieurs mouvements qu'il juge optimaux et qui permettront le calcul de la référence la plus appropriée pour le patient. L'ensemble de ces mouvements enregistrés permet également de calculer des bornes dynamiques d'exécution sur l'ensemble des paramètres du mouvement à contrôler à l'intérieur desquelles le mouvement sera jugé correct. Le thérapeute peut également décider de fixer ces bornes a priori. Pour un même mouvement optimal, il peut exister différentes bornes présentant des amplitudes décroissantes correspondant à une augmentation de la difficulté de l'exercice au fur et à mesure de la progression de l'individu.

On notera que le spécialiste peut définir la marge d'erreur pour un paramètre particulier du mouvement, selon l'objectif d'amélioration donné à l'individu. Par exemple, dans le cas de la marche, un tel paramètre peut être : la vitesse d'exécution du mouvement, l'amplitude du mouvement, le rythme de pose d'un talon sur le sol, la symétrie (liste non limitative). Ainsi, la rétroaction pourra être déclenchée spécifiquement en cas d'écart par rapport audit paramètre et non nécessairement par rapport au mouvement considéré dans son ensemble.

De plus, le système est capable de conserver l'historique d'un nombre défini des derniers essais, ce qui permet aux bornes préalablement fixées autour du mouvement optimal d'évoluer automatiquement au cours du temps en fonction des progrès effectués par l'individu. Le système est donc rendu adaptatif à l'évolution du mouvement de l'individu.

Naturellement, au fur et à mesure de l'entraînement de l'individu et de l'évolution de ses capacités, le thérapeute peut également être amené à modifier le mouvement optimal et/ou à affiner les bornes autour du mouvement optimal.

Le processeur étant solidaire de l'individu (par exemple intégré à un vêtement ou intégré à un smartphone embarqué sur l'individu), l'individu bénéficie d'une pleine liberté de ses mouvements, puisqu'il n'a pas besoin d'être relié à un ordinateur qui réaliserait l'enregistrement ou la comparaison des mouvements. De manière avantageuse, la liaison entre les capteurs et le processeur est réalisée sans fil afin de conserver cette liberté de mouvement.

Le mouvement étant cyclique, le système est capable automatiquement de reconnaître le mouvement à contrôler lorsque ce dernier se répète un certain nombre de fois consécutives, et de redevenir inactif à partir du moment où l'utilisateur change d'activité. Il est bien sûr entendu que plusieurs mouvements cycliques peuvent être enregistrés et le système sera alors capable de les reconnaître afin d'utiliser le mouvement de référence correspondant.

Le système comprend en outre au moins un dispositif de rétroaction couplé au processeur et destiné à fournir à l'individu une information sur la conformité du mouvement vis-à-vis du mouvement de référence. Cette information peut être fournie sous la forme d'un signal visuel, sonore et/ou tactile.

Par exemple, des bornes dynamiques étant définies au préalable, le mouvement est considéré comme correct (c'est-à-dire conforme au mouvement de référence) si le résultat de l'ensemble des comparaisons entre les paramètres du mouvement de référence et les paramètres du mouvement en cours d'exécution est entre lesdites bornes. Au contraire, si le résultat de la comparaison est en dehors de ces bornes pendant une durée définie au préalable, le processeur transmet au dispositif de rétroaction un ordre d'émission d'un signal qui peut être différent en fonction de la nature de l'erreur calculée.

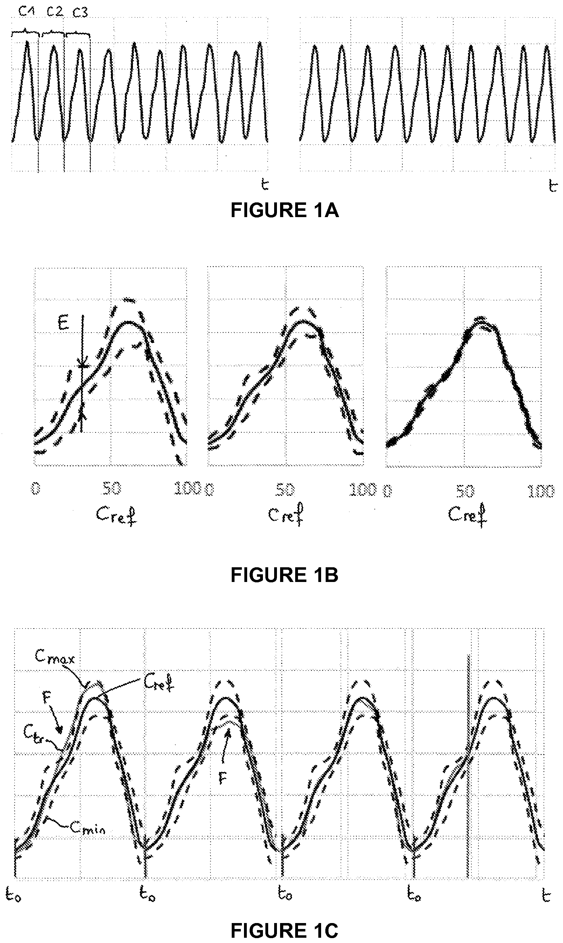

La figure 1A illustre l'enregistrement des données d'un mouvement cyclique déterminé.

Dans cette étape, l'individu est équipé du ou des capteurs tridimensionnels de mouvement. Sous le contrôle d'un spécialiste (par exemple un thérapeute, un ergonome), il réalise le mouvement cyclique au mieux de ses capacités actuelles. On enregistre ainsi un mouvement considéré comme acceptable puis on choisit ensuite le ou les paramètres du mouvement à contrôler (la courbe de gauche représentant un tel paramètre en fonction du temps t), chaque cycle étant désigné par le repère C₁, C₂, C₃, etc. La courbe de droite représente les données idéales de ce paramètre du mouvement, le mouvement pouvant être soit réalisé par un autre individu sain, soit par l'individu lui-même à un stade ultérieur de son traitement, traduisant une progression dans l'accomplissement du mouvement. Eventuellement, il peut exister un certain nombre d'enregistrements intermédiaires (non représentés) au fur et à mesure des progrès de l'individu.

La figure 1B illustre le calcul d'un cycle de référence C_{ref} à partir de l'enregistrement représenté à la figure 1A, correspondant à une moyenne arithmétique de la durée d'un certain nombre de cycles, ainsi que le calcul de l'erreur E admise pour ledit cycle C_{ref}.

Dans l'exemple illustré à la figure 1B, il existe trois niveaux de difficulté correspondant à une marge d'erreur progressivement décroissante : la courbe de gauche illustre une marge d'erreur élevée, correspondant à un exercice facile, la courbe de droite illustre une marge d'erreur minimale, correspondant à un exercice difficile, et la courbe du milieu illustre une marge d'erreur intermédiaire, correspondant à un exercice de difficulté moyenne. Ici, la marge d'erreur est représentée par des pointillés sur l'ensemble du mouvement (c'est-à-dire entre 0 et 100% du cycle), mais cette dernière peut n'être définie qu'en un instant, ou que sur une partie du mouvement également.

Le processeur peut appliquer dans un premier temps la marge élevée puis, une fois que le mouvement de l'individu est conforme à ladite marge, le processeur applique la marge d'erreur intermédiaire tant que le mouvement de l'individu n'est pas conforme à ladite marge et enfin, le processeur applique la marge d'erreur minimale. Une fois que l'individu est capable de reproduire le mouvement acceptable en respectant ladite marge d'erreur minimale sur le ou les paramètres du mouvement qui sont contrôlés, le mouvement est ajusté de manière à tendre vers le mouvement idéal, les marges d'erreur précédemment définies étant appliquées à ce mouvement ajusté.

La figure 1C illustre schématiquement la mise en oeuvre du procédé dans la vie quotidienne de l'individu.

L'individu est équipé du ou des capteurs tridimensionnels de mouvement qui sont ajustés sur le ou les segments corporels concernés et qui, grâce au vêtement qui permet de les solidariser à l'individu, sont quasiment invisibles pour les tiers.

Lorsque l'individu commence à effectuer un mouvement cyclique enregistré, le processeur utilise les premiers cycles pour détecter ledit mouvement et pour calculer la durée moyenne du cycle. En effet, on considère qu'un mouvement cyclique donné présente toujours une même organisation au cours du temps. Par conséquent, même si le mouvement est réalisé à une vitesse différente de celle du mouvement enregistré, l'organisation de ce mouvement reste identique et il suffit que le processeur applique la durée moyenne mesurée pour étalonner le cycle de référence enregistré.

Par exemple, s'agissant du mouvement de la marche, celui-ci est constitué de deux phases principales :
(I) la phase d'appui qui correspond à la période où au moins une partie du pied est en contact avec le sol, qui s'étend typiquement entre 0 et 60% du cycle de marche, et qui se divise en trois phases secondaires :
   (1.1) la phase taligrade, qui débute avec le contact initial du talon avec le sol et se poursuit par la mise en charge du membre inférieur droit ; ladite phase s'étend entre 0 et 10% du cycle de marche ;
   (I.2) la phase plantigrade, qui débute lorsque le pied repose sur la plante du pied et s'achève lorsque le talon perd contact avec le sol ; ladite phase s'étend entre 10 et 50% du cycle de marche ;
   (I.3) la phase digitigrade, qui débute lorsque le talon est levé et se termine lorsque le pied a décollé du sol ; ladite phase s'étend entre 50 et 60% du cycle de marche ;
(II) la phase oscillante où le pied n'est plus en contact avec le sol et qui permet l'avancée du membre inférieur, qui s'étend entre 60 et 100% du cycle de marche, et qui est divisée en deux phases secondaires :
   (II.1) la phase de raccourcissement de la jambe ;
   (II.2) la phase d'allongement de la jambe.

Ensuite, au fur et à mesure de l'accomplissement du mouvement par l'individu (représenté par la courbe Cₜᵣ) au cours du temps t, le processeur détecte chaque début de cycle (noté to) et applique la marge d'erreur prédéterminée (schématisée par les courbes Cₘᵢₙ et Cₘₐₓ qui entourent le cycle de référence C_{ref}).

Lorsque le mouvement réalisé sort de la marge d'erreur, le processeur déclenche une rétroaction (notée F) pour informer l'individu de l'erreur et l'inciter à corriger son mouvement. Ainsi, sur le premier cycle, une rétroaction F est déclenchée car le mouvement enregistré Cₜᵣ dépasse la borne Cₘₐₓ ; sur le deuxième cycle, une rétroaction F est déclenchée car le mouvement enregistre Cₜᵣ passe en-deçà de la borne Cₘᵢₙ, tandis qu'aucune rétroaction n'est déclenchée sur le troisième cycle car le mouvement enregistré reste à l'intérieur de l'intervalle [Cₘᵢₙ, Cₘₐₓ]. Comme on le voit sur la figure 1C, la rétroaction est déclenchée en temps réel, c'est-à-dire dès qu'une sortie de la marge d'erreur est mesurée pour le ou les paramètres du mouvement considérés. Ainsi, l'individu peut modifier l'exécution du mouvement en temps réel, dès le cycle considéré, sans attendre d'avoir terminé un exercice complet.

De préférence, le dispositif de rétroaction peut être rendu solidaire de l'individu par tout moyen approprié.

Tel est le cas notamment lorsque le signal de rétroaction est tactile. Par exemple, le dispositif de rétroaction comprend au moins une électrode ou un vibreur maintenu en contact avec la peau de l'individu. Si le(s) capteur(s) et éventuellement le processeur sont intégrés à un vêtement, le dispositif de rétroaction peut également être intégré audit vêtement.

Un seul dispositif de rétroaction peut être suffisant pour donner à l'individu une information selon laquelle le mouvement est conforme ou non au mouvement de référence. Eventuellement, plusieurs dispositifs de rétroaction peuvent être utilisés et répartis de manière à fournir à l'individu une information plus précise sur la portion de mouvement non conforme au mouvement de référence, le segment n'ayant pas réalisé un mouvement conforme au mouvement de référence ou sur le paramètre du mouvement qui n'est pas conforme. Eventuellement, l'intensité du signal tactile peut être modulée en fonction de l'erreur constatée.

Dans le cas d'un signal visuel, le dispositif de rétroaction peut par exemple comprendre une lampe qui s'allume lorsque le résultat de la comparaison est en-dehors des bornes prédéfinies. Ladite lampe peut être rendue solidaire de l'individu par tout moyen approprié, tel qu'un vêtement comme mentionné plus haut.

De manière alternative, le dispositif de rétroaction peut comprendre l'écran d'un smartphone, qui affiche une information visuelle.

Dans le cas d'un signal sonore, le dispositif de rétroaction peut comprendre un haut-parleur. Ledit haut-parleur peut être solidaire ou non de l'individu.

De manière alternative, le dispositif de rétroaction peut être inclus dans un appareil distinct du vêtement, de préférence un appareil portatif tel qu'un téléphone portable ou un assistant portatif personnel. Dans ce cas, le dispositif de rétroaction est couplé au processeur par une technologie sans fil, selon un protocole de communication approprié. La rétroaction peut par exemple consister à symboliser le ou les segments impliqués dans le mouvement et à afficher dans une couleur différente le ou les segments en cause lorsque le mouvement n'est pas conforme au mouvement de référence, sur l'écran de l'appareil. De manière alternative ou complémentaire, la rétroaction peut également consister en une vibration de l'appareil signalant une non-conformité du mouvement vis-à-vis du mouvement de référence. Ceci permet en particulier une rétroaction perceptible uniquement par l'individu et non par son entourage.

La rétroaction peut être réalisée en temps réel, par exemple dans le cas d'un dispositif tactile, pour permettre à l'individu de se rendre compte de la non-conformité du mouvement dès que celle-ci est constatée. De manière alternative, la comparaison du mouvement enregistré et du mouvement de référence peut être mémorisée de manière à pouvoir être rejouée ultérieurement.

Dans tous les cas, la rétroaction vise uniquement à fournir une information à l'individu pour conduire celui-ci à corriger lui-même son mouvement, et non à corriger par des actuateurs le mouvement réalisé par l'individu. Ainsi, l'individu est pleinement actif dans la démarche de correction du mouvement, qui est donc plus efficace.

D'autre part, le fait que le mouvement de référence soit propre à l'individu, en tenant compte de ses capacités physiques initiales et de leur évolution, et non externe à celui-ci (comme le serait un mouvement idéal standard) permet à l'individu d'atteindre plus facilement l'objectif fixé et rend cet apprentissage plus efficace.

Par ailleurs, le processeur peut mémoriser l'historique des mises en oeuvre successives du mouvement, pour représenter les échecs et succès de l'individu au cours de son apprentissage. A cet égard, le cas où le ou les capteurs sont solidaires d'un vêtement est avantageux dans la mesure où ce mode de réalisation assure un emplacement constant de chaque capteur par rapport à un segment, de sorte que les différents essais sont répétables.

Le système comprend en outre une batterie permettant l'alimentation électrique du ou des capteurs, du processeur et, le cas échéant, du ou des dispositifs de rétroaction. Pour minimiser la nécessité de recharger ou remplacer la batterie, on emploie de préférence des éléments dont la consommation électrique est minimale.

L'intégration des différents éléments au vêtement peut se faire par tout moyen de solidarisation approprié, par exemple par collage sur la surface interne ou externe du vêtement, par incorporation aux fibres du textile lors de sa fabrication, par coutures, etc.

En raison de la flexibilité du matériau textile du vêtement, celui-ci n'entrave pas les gestes de l'individu et présente donc un certain confort d'utilisation. De plus, le vêtement présentant une faible épaisseur et épousant la forme du corps, il est relativement discret et peut éventuellement être porté sous un autre vêtement, de sorte que d'autres personnes que l'individu ne perçoivent pas sa fonction technique. Pour favoriser le confort du vêtement, les différents éléments sont avantageusement miniaturisés de sorte à présenter une masse et un encombrement les plus faibles possibles.

En fonction de la pathologie et/ou de l'individu, des postures ou des mouvements erronés peuvent impliquer différents systèmes sensoriels, principalement le système visuel, le système proprioceptif et/ou le système vestibulaire.

Pour identifier le système en cause dans la non-conformité du mouvement, les capteurs sont avantageusement choisis et agencés dans le vêtement pour permettre de déterminer la contribution de chaque système au mouvement.

Ainsi, la contribution du système proprioceptif peut être estimée en comparant la position et/ou l'orientation d'un segment lors du mouvement par rapport au mouvement de référence. A cet effet, les capteurs tridimensionnels positionnés sur les segments particuliers dont on veut évaluer la proprioception sont utilisés.

La contribution du système vestibulaire peut être estimée en évaluant l'équilibre de l'individu au regard de l'accélération linéaire ou angulaire d'un ou plusieurs capteurs. A cet effet, au moins un capteur tridimensionnel ou simplement un accéléromètre situé sur la tête est utilisé. Il est possible d'affiner cette mesure avec d'autres capteurs situés par exemple sur le bassin ou le haut du corps pour apprécier l'équilibre général du sujet.

La contribution du système visuel peut être estimée en évaluant la position de la tête par rapport aux épaules. A cet effet, un capteur tridimensionnel situé sur la tête et un autre situé sur une épaule ou dans le haut du dos sont utilisés..

En fonction du système identifié comme défaillant lors de l'exécution du mouvement, le ou les dispositifs de rétroaction peuvent être agencés de sorte à indiquer à l'utilisateur la nature de l'erreur et son emplacement.

L'invention peut trouver des applications dans de nombreux domaines. Parmi ceux-ci, on peut citer notamment la rééducation ou l'ergonomie. Hors du domaine médical, on peut citer le domaine sportif et plus généralement toutes les situations d'apprentissage qui impliquent la motricité et la répétition des gestes (entraînement de l'individu visant à améliorer un geste).

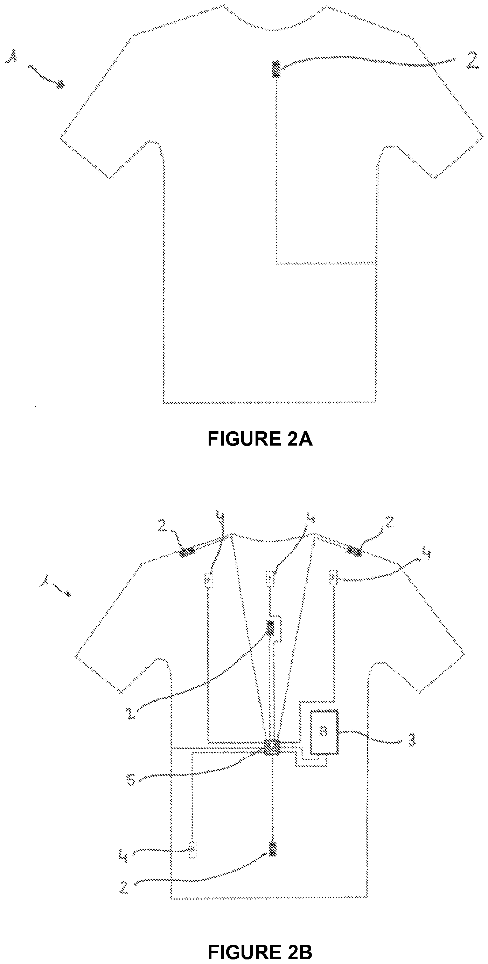

Les figures 2A et 2B illustrent un mode de réalisation de l'invention, respectivement en vue de face et en vue de dos.

Dans cet exemple, le vêtement 1 est un T-shirt réalisé dans un matériau textile extensible, de manière à pouvoir être étroitement ajusté à la partie haute du corps d'un individu.

Le vêtement comprend plusieurs capteurs 2, à savoir :
- trois capteurs dans le plan sagittal : un capteur sur l'avant du vêtement, à proximité de l'encolure, et deux capteurs sur le dos du vêtement, respectivement dans le haut et dans le bas du dos ;
- deux capteurs agencés chacun sur une épaule dans le dos du vêtement, de manière symétrique par rapport au plan sagittal. Dans ce cas, le segment corporel dont on étudie le mouvement est le tronc de l'individu.

Dans ce cas, les capteurs sont des centrales inertielles agencées de sorte à rendre compte des mouvements du torse de l'individu dans les trois dimensions.

Le vêtement 1 comprend en outre quatre dispositifs de rétroaction 4 (par exemple des électrodes ou des vibreurs) : trois de ces dispositifs sont agencés dans un plan horizontal au dos du vêtement, et un est agencé au bas du dos, à distance du plan sagittal.

Le vêtement 1 comprend par ailleurs un processeur 5 couplé à la fois aux capteurs 2 et aux dispositifs de rétroaction 4. Eventuellement, s'il y a un grand nombre de capteurs, le vêtement peut comprendre plusieurs processeurs. De manière alternative, le processeur pourrait être distinct du vêtement et appartenir, par exemple, à un smartphone embarqué sur l'individu.

Dans cet exemple, les liaisons entre le processeur, les capteurs et les dispositifs de rétroaction sont représentées sous forme filaire, mais elles pourraient éventuellement être réalisées sans fil au moyen d'un protocole de communication adéquat.

Enfin, le vêtement 1 comprend en outre une batterie 3 destinée à alimenter en énergie le processeur 5, les capteurs 2 et les dispositifs de rétroaction 4.

La solidarisation des capteurs, des dispositifs de rétroaction, du processeur et de la batterie au vêtement peut être réalisée par tout moyen. Parmi les moyens de solidarisation, on peut citer le collage, la couture, l'intégration au procédé de fabrication (par exemple tissage) du matériau textile, etc.

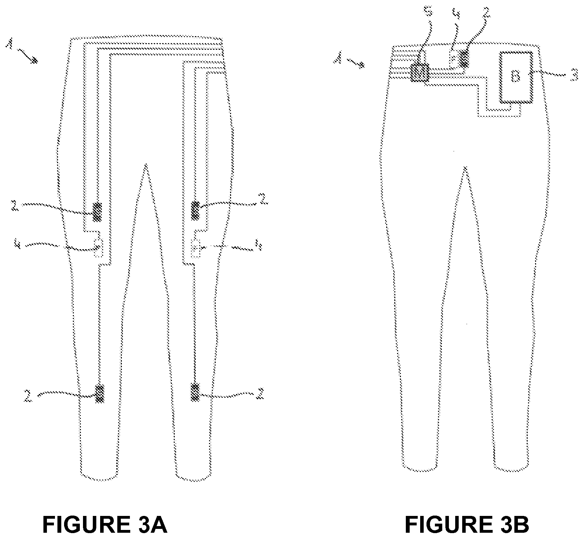

Les figures 3A et 3B illustrent un autre mode de réalisation de l'invention, respectivement en vue de face et en vue de dos.

Dans cet exemple, le vêtement 1 est un pantalon réalisé dans un matériau textile extensible, de manière à pouvoir être étroitement ajusté à la partie basse du corps d'un individu.

Les signes de référence repris de la figure 1 correspondent aux mêmes éléments.

Dans ce cas, le vêtement 1 comprend deux capteurs sur l'avant de chaque jambe, respectivement au-dessus du genou et à mi-mollet, et un capteur situé au dos, à proximité de la ceinture. Les segments auxquels on s'intéresse sont les parties inférieure et supérieure de chaque jambe.

Le vêtement 1 comprend en outre un dispositif de rétroaction 4 situé sur l'avant de chaque genou.

Le processeur 5 et la batterie 3 sont agencés dans le dos du vêtement, à proximité de la ceinture.

Enfin, il va de soi que les exemples que l'on vient de donner ne sont que des illustrations particulières en aucun cas limitatives quant aux domaines d'application de l'invention.

### REFERENCES

[1] WO 2009/112281
[2] US 2011/0063114
[3] US 2013/0184611
[4] US 2013/0207889
[5] US 2014/303529
[6] WO 2008/129442
[7] Williams MJ, Haq I, Raymond YL, Dynamic measurement of lumbar curvature using fibre-optic sensors, Medical Engineering and Physics, 2010
[8] Tognetti A, Lorussi F, Dalle Mura G, Carbonaro N, Pacelli M, Paradiso R, De Rossi D, New génération of wearable goniometers for motion capture systems, Journal of NeuroEngineering and Réhabilitation, 2014

## Revendications

1. Système de contrôle d'un mouvement cyclique d'au moins un segment corporel d'un individu, comprenant :
- au moins un capteur (2) tridimensionnel de mouvement,
- un moyen de fixation dudit capteur sur ledit segment corporel de l'individu,
- un dispositif de rétroaction (4) ;
- un processeur (5) couplé audit capteur (2) et apte à être embarqué sur l'individu, ledit processeur étant configuré pour
enregistrer un mouvement cyclique de référence propre à l'individu et des bornes de tolérance minimales et maximales pour un paramètre du mouvement à contrôler, les bornes de tolérance étant propres à l'individu et évolutives en fonction de sa progression, les bornes de tolérance du paramètre de mouvement étant des bornes dynamiques de sorte à définir une courbe de valeurs minimales et une courbe de valeurs maximales entourant la courbe suivie par le paramètre de mouvement du mouvement cyclique de référence;
détecter, au moyen du capteur, ledit mouvement cyclique du segment corporel;
à partir d'une pluralité de cycles du mouvement cyclique détecté, calculer la durée moyenne d'un cycle et étalonner le mouvement cyclique de référence avec ladite durée moyenne ;
à partir de données de mesure dudit capteur, calculer ledit paramètre pour le mouvement détecté ;
comparer en temps réel, au moyen d'un algorithme de comparaison points par points de signaux, ledit paramètre pour le mouvement détecté (Cₜᵣ) avec ledit paramètre du mouvement cyclique de référence (C_{ref}) étalonné afin de déterminer si le résultat de la comparaison est entre les bornes de tolérance ; et
déclencher une rétroaction par le dispositif de rétroaction lorsque le ledit paramètre pour le mouvement détecté sort de la marge d'erreur définie par les bornes de tolérance ;
- le dispositif de rétroaction (4) étant couplé audit processeur (5) et adapté pour fournir à l'individu une information en temps réel sur la conformité du paramètre du mouvement détecté vis-à-vis du paramètre dudit mouvement cyclique de référence.

2. Système selon la revendication 1, dans lequel le moyen de fixation du capteur sur le segment corporel est un vêtement dont au moins une partie est ajustable audit segment corporel, le capteur étant solidaire dudit vêtement.

3. Système selon la revendication 2, dans lequel le dispositif de rétroaction est inclus dans un appareil portatif distinct du vêtement et est relié au vêtement (5) par une liaison sans fil.

4. Système selon l'une des revendications 2 ou 3, dans lequel le processeur (5) est solidaire du vêtement (1).

5. Système selon l'une des revendications 2 ou 3, dans lequel le processeur (5) est distinct du vêtement (1).

6. Système selon l'une des revendications 1 ou 5, dans lequel le dispositif de rétroaction (4) est configuré pour émettre un signal sonore.

7. Système selon l'une des revendications 1 à 6, dans lequel le dispositif de rétroaction (4) est configuré pour émettre un signal visuel.

8. Système selon l'une des revendications 1 à 7, dans lequel le dispositif de rétroaction (4) est configuré pour émettre un signal tactile.

9. Système selon l'une des revendications 1 à 8, dans lequel le dispositif de rétroaction (4) comprend au moins un vibreur, une électrode, un haut-parleur, et/ou un écran affichant une représentation du mouvement.

10. Système selon l'une des revendications 1 à 9, dans lequel le capteur tridimensionnel (2) est une centrale inertielle.

11. Système selon l'une des revendications 1 à 10, comprenant au moins deux capteurs tridimensionnels destinés à être rendus solidaires d'un même segment corporel, le processeur étant configuré pour fusionner les données de mesure desdits capteurs.

12. Système selon l'une des revendications 1 à 11, dans lequel le processeur (5) est couplé au capteur (2) par une liaison sans fil.

13. Système selon l'une des revendications 1 à 12, dans lequel le processeur est configuré pour, après l'exécution d'une pluralité de cycles conformes au mouvement de référence, faire évoluer automatiquement au cours du temps les bornes de tolérances de sorte à diminuer la marge de tolérance et/ou ajuster le mouvement cyclique de référence.

## Patentansprüche

1. System zur Steuerung einer zyklischen Bewegung mindestens eines Körpersegments einer Person, umfassend:
- mindestens einen dreidimensionalen Bewegungssensor (2),
- ein Mittel zum Befestigen des Sensors an dem Körpersegment der Person,
- eine Rückkopplungsvorrichtung (4);
- einen Prozessor (5), der mit dem Sensor (2) gekoppelt ist und geeignet ist, um an der Person angebracht zu werden, wobei der Prozessor konfiguriert ist zum
Aufzeichnen einer zyklischen Referenzbewegung, die für die Person typisch ist, und von minimalen und maximalen Toleranzgrenzen für einen zu steuernden Bewegungsparameter, wobei die Toleranzgrenzen für die Person typisch sind und sich in Abhängigkeit von ihrem Fortschreiten entwickeln, wobei die Toleranzgrenzen des Bewegungsparameters dynamische Grenzen sind, um eine Kurve von minimalen Werten und eine Kurve von maximalen Werten zu definieren, die die Kurve umgeben, der der Bewegungsparameter der zyklischen Referenzbewegung folgt;
Erfassen, mittels des Sensors, der zyklischen Bewegung des Körpersegments;
ausgehend von einer Vielzahl von Zyklen der erfassten zyklischen Bewegung, Berechnen der mittleren Dauer eines Zyklus und Kalibrieren der zyklischen Referenzbewegung mit der mittleren Dauer;
ausgehend von den Messdaten des Sensors, Berechnen des Parameters für die erfasste Bewegung;
Vergleichen in Echtzeit, mittels eines Punkt-für-Punkt-Signalvergleichsalgorithmus, des Parameters für die erfasste Bewegung (Ctr) mit dem Parameter der kalibrierten zyklischen Referenzbewegung (C_{ref}), um zu bestimmen, ob das Ergebnis des Vergleichs zwischen den Toleranzgrenzen liegt; und
Auslösen einer Rückkopplung durch die Rückkopplungsvorrichtung, wenn der Parameter für die erfasste Bewegung den Fehlerbereich verlässt, der durch die Toleranzgrenzen bestimmt ist;
- wobei die Rückkopplungsvorrichtung (4) mit dem Prozessor (5) gekoppelt ist und ausgelegt ist, um der Person Informationen in Echtzeit über die Übereinstimmung des Parameters der erfassten Bewegung mit dem Parameter der zyklischen Referenzbewegung bereitzustellen.

2. System nach Anspruch 1, wobei das Mittel zum Befestigen des Sensors an dem Körpersegment ein Kleidungsstück ist, von dem mindestens ein Teil an das Körpersegment anpassbar ist, wobei der Sensor mit dem Kleidungsstück fest verbunden ist.

3. System nach Anspruch 2, wobei die Rückkopplungsvorrichtung in eine tragbare Einrichtung eingeschlossen ist, die von dem Kleidungsstück verschieden ist und mit dem Kleidungsstück (5) durch eine drahtlose Verbindung verbunden ist.

4. System nach einem der Ansprüche 2 oder 3, wobei der Prozessor (5) mit dem Kleidungsstück (1) fest verbunden ist.

5. System nach einem der Ansprüche 2 oder 3, wobei der Prozessor (5) von dem Kleidungsstück (1) verschieden ist.

6. System nach einem der Ansprüche 1 oder 5, wobei die Rückkopplungsvorrichtung (4) zum Aussenden eines Schallsignals konfiguriert ist.

7. System nach einem der Ansprüche 1 oder 6, wobei die Rückkopplungsvorrichtung (4) zum Aussenden eines visuellen Signals konfiguriert ist.

8. System nach einem der Ansprüche 1 oder 7, wobei die Rückkopplungsvorrichtung (4) zum Aussenden eines taktilen Signals konfiguriert ist.

9. System nach einem der Ansprüche 1 bis 8, wobei die Rückkopplungsvorrichtung (4) mindestens einen Vibrator, eine Elektrode, einen Lautsprecher und/oder einen Bildschirm umfasst, der eine Darstellung der Bewegung anzeigt.

10. System nach einem der Ansprüche 1 oder 9, wobei der dreidimensionale Sensor (2) eine Trägheitsmesseinheit ist.

11. System nach einem der Ansprüche 1 bis 10, umfassend mindestens zwei dreidimensionale Sensoren, die dafür bestimmt sind, mit einem gleichen Körpersegment fest verbunden zu werden, wobei der Prozessor zum Zusammenführen der Messdaten der Sensoren konfiguriert ist.

12. System nach einem der Ansprüche 1 oder 11, wobei der Prozessor (5) mit dem Sensor (2) durch eine drahtlose Verbindung gekoppelt ist.

13. System nach einem der Ansprüche 1 bis 12, wobei der Prozessor zum, nach der Ausführung einer Vielzahl von Zyklen, die mit der Referenzbewegung übereinstimmen, automatischen Entwickeln im Laufe der Zeit der Toleranzgrenzen konfiguriert ist, um den Toleranzbereich zu verringern und/oder die zyklische Referenzbewegung anzupassen.

## Claims

1. A system for controlling a cyclic movement of at least one body segment of an individual, comprising:
- at least one three-dimensional movement sensor (2);
- a means for fastening said sensor to said body segment of the individual;
- a feedback device (4); and
- a processor (5) which is coupled to said sensor (2) and is capable of being carried on the individual, said processor being configured to
record a reference cyclic movement specific to the individual and minimum and maximum tolerance limits for a parameter of the movement to be monitored, the tolerance limits being specific to the individual and changing depending on their progress, the tolerance limits of the movement parameter being dynamic limits so as to define a curve of minimum values and a curve of maximum values surrounding the curve followed by the movement parameter of the reference cyclic movement;
detect, by means of the sensor, said cyclic movement of the body segment;
from a plurality of cycles of the detected cyclic movement, calculate the average duration of a cycle and calibrate the reference cyclic movement with said average duration;
from measurement data of said sensor, calculate said parameter for the detected movement;
compare, in real time, by means of a signal point-to-point comparison algorithm, said parameter for the detected movement (Ctr) with said parameter of the reference cyclic movement (C_{ref}) calibrated to determine whether the result of the comparison is between the tolerance limits; and
trigger feedback via the feedback device when said parameter for the detected movement
goes outside the margin of error defined by the tolerance limits;
- the feedback device (4) being coupled to said processor (5) and being suitable for providing the individual with real-time information on the compliance of the parameter of the detected movement with respect to the parameter of said reference cyclic movement.

2. The system according to claim 1, wherein the means for fastening the sensor to the body segment is a garment, at least part of which can be adjusted to said body segment, the sensor being secured to said garment.

3. The system according to claim 2, wherein the feedback device is included in a portable apparatus which is separate from the garment and is connected to the garment (5) via a wireless connection.

4. The system according to either claim 2 or claim 3, wherein the processor (5) is secured to the garment (1).

5. The system according to either claim 2 or claim 3, wherein the processor (5) is separate from the clothing (1).

6. The system according to either claim 1 or claim 5, wherein the feedback device (4) is configured to emit a sound signal.

7. The system according to any of claims 1 to 6, wherein the feedback device (4) is configured to emit a visual signal.

8. The system according to any of claims 1 to 7, wherein the feedback device (4) is configured to emit a tactile signal.

9. The system according to any of claims 1 to 8, wherein the feedback device (4) comprises at least one vibrator, electrode, loudspeaker and/or screen displaying a representation of the movement.

10. The system according to any of claims 1 to 9, wherein the three-dimensional sensor (2) is an inertial unit.

11. The system according to any of claims 1 to 10, comprising at least two three-dimensional sensors which are intended to be secured to the same body segment, the processor being configured to merge the measurement data of said sensors.

12. The system according to any of claims 1 to 11, wherein the processor (5) is coupled to the sensor (2) via a wireless connection.

13. The system according to any of claims 1 to 12, wherein the processor is configured, after the execution of a plurality of cycles in accordance with the reference movement, to change the tolerance limits automatically over time so as to reduce the tolerance margin and/or to adjust the reference cyclic movement.
